# EUROPEAN PATENT SPECIFICATION

(11) **EP 1 333 838 B1**
(45) Date of publication and mention of the grant of the patent: **13.12.2006**
(21) Application number: 01975935.6
(22) Date of filing: 25.10.2001
(51) Int. Cl.: A61K 31/58, A61P 3/04, A61K 31/683

(54) **COMPOUNDS COMPRISING A PHYTOSTEROL AND/OR PHYTOSTANOL MOIETY AND ASCORBIC ACID AND USE THEREOF AS WEIGHT REGULATING AGENTS**
VERBINDUNGEN DIE EIN PHYTOSTEROL- UND/ODER EIN PHYTOSTANOLTEIL UND ASCORBINSÄURE ENTHALTEN UND DEREN VERWENDUNG ALS GEWICHTSREGULATOREN
COMPOSES COMPRENANT DU PHYTOSTEROL ET/OU DU PHYTOSTANOL ET DE L'ACIDE ASCORBIQUE ET LEUR UTILISATION COMME AGENTS DE REGULATION DU POIDS

(30) Priority: 25.10.2000 US 985749
(43) Date of publication of application: 13.08.2003
(73) Proprietor: Forbes Medi-Tech Inc., Vancouver, British Columbia V6C 2T8 (CA)
(72) Inventor: KUTNEY, James, P., Vancouver, British Columbia V6L 3C7 (CA); MILANOVA, Radka, K., Vancouver, British Columbia V6G 1E1 (CA); PRITCHARD, Haydn, P., Vancouver, British Columbia V6N 2X6 (CA); NOVAK, Egon, Richmond, British Columbia V6X 3T1 (CA); LUKIC, Tatjana, Vancouver, British Columbia V6E 1E2 (CA)
(74) Representative: Jones, Helen M.M.
(86) International application number: PCT/CA2001/001483
(87) International publication number: WO 2002/034241

(56) References cited:
- EP-A- 0 339 486
- EP-A- 0 436 936
- EP-A- 0 665 238
- WO-A-01/00653
- WO-A-97/42960

## Description

### FIELD OF THE INVENTION

This present invention relates to the field of sterol and stanol derivatives and their use in decreasing weight gain and increasing weight loss in animals, including humans.

### BACKGROUND OF THE INVENTION

While recent advances in science and technology are helping to improve quality and add years to human life, the issue of maintaining and controlling a healthy body weight and the impact of weight on overall health and longevity is only recently receiving probative attention from the medical community.

Sterols are naturally occurring compounds that perform many critical cellular functions. Phytosterols such as campesterol, stigmasterol and beta-sitosterol in plants, ergosterol in fungi and cholesterol in animals are each primary components of cellular and sub-cellular membranes in their respective cell types. The dietary source of phytosterols in humans comes from plant materials i.e. vegetables and plant oils. The estimated daily phytosterol content in the conventional western-type diet is approximately 60-80 milligrams in contrast to a vegetarian diet which would provide about 500 milligrams per day.

Phytosterols have received a great deal of attention due to their ability to decrease serum cholesterol levels when fed to a number of mammalian species, including humans. While the precise mechanism of action remains largely unknown, the relationship between cholesterol and phytosterols is apparently due in part to the similarities between the respective chemical structures (the differences occurring in the side chains of the molecules). It is assumed that phytosterols displace cholesterol from the micellar phase and thereby reduce its absorption or possibly compete with receptor and/or carrier sites in the cholesterol absorption process.

The advantages of phytosterols, not only in the treatment of CVD and its underlying conditions such as hypercholesterolemia, hyperlipidemia, atherosclerosis, hypertension, thrombosis but in the treatment of other diseases such as Type II diabetes, dementia cancer and aging, are well recorded.

Many compositions for use in weight control have been identified in the prior art.

EP-A-0665238 relates to (1) corticoid derivatives of a formula or physiologically acceptable salts thereof (hereinafter referred to collectively as the present compound), (2) processes for producing the present compound, and (3) useful compositions containing the present compound. The corticoid that can be used in this invention is any corticosteroid having an alcoholic hydroxyl group or a halogen atom in 21- position of the steroid skeleton. Mineralocorticoids, aldosterone, desoxycorticosterone, corticosterone are mentioned. Phytosterols and phytostanols are not mentioned.

WO97/42960 describes a method for coupling a molecule of L-ascorbic acid to a molecule of cholesterol through a bioreversible phosphate linkage at position 2 or 3 on the ascorbyl group and position 31 on the cholesteryl moiety. These derivatives are noted to be stable, easily incorporated into cosmetically acceptable vehicles and enzymatically bioreversible in the skin to free ascorbic acid and a safe cholesterol component. An exemplary embodiment is 3'(L-ascorbyl-2-phosphoryl)cholesterol. There is no teaching of the combination of phytosterols and phytostanols to an ascorbyl phosphate moiety for the purpose of reducing body weight.

EP-A-0436936 provides lipid-selective antioxidants of the general formula I (A)a(L)(X)a(I) in which A = antioxidant component, L = linker, X = lipophilic component, a and a' = independently of one another the numbers 1 or 2. The compounds are used to protect lipid-containing substances from oxidation and in pharmaceutical compositions for the prophylaxis and therapy of diseases in which bioradicals are involved, in particular diseases of the heart, circulation and vessels. There is no teaching of the combination of phytosterols and/or phytostanols to an ascorbyl phosphate moiety for the purpose of reducing body weight.

WO01/00653 discloses use of phytosterol derivatives, phytostanol derivatives, and ascorbic acid derivatives for treating cardiovascular disease including atherosclerosis and hyperlipidemia. Other disclosed uses of the compounds include hypertension, thrombosis, Type II diabetes, dementia, Alzheimers disease, ageing and cancer.

It is an object of the present invention to modify phytosterols and phytostanols for use in the field of controlling and regulating weight in animals, particularly in humans and to obviate and mitigate the problems associated with some of the currently available weight loss products.

### SUMMARY OF THE INVENTION

The present invention provides a novel use of one or more of the structures having the following formulae: 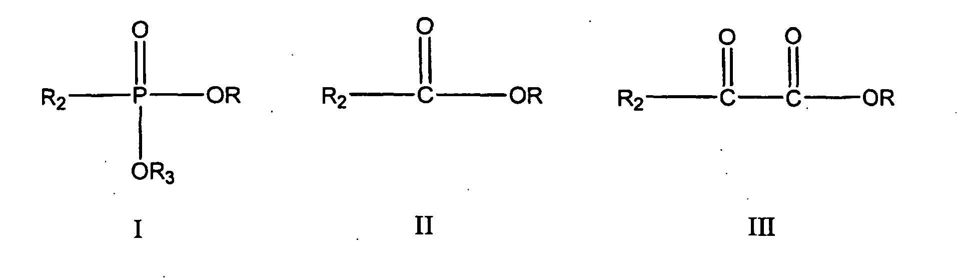 wherein R is a sterol or stanol moiety, R₂ is an ascorbic acid moiety and R₃ is hydrogen or any metal, alkali earth metal or alkali metal; and all salts of these structures in the manufacture of a composition for use in a method of weight control.

The animal is preferably a human.

The method of weight control may result in a decrease in weight gain or an increase in weight loss.

The composition may be a comestible or beverage. Accordingly foods, beverages and nutraceuticals may be supplemented with derivatives of sterols and/of stanols and ascorbic acid, having one or more of the above noted formulae, for use in the present invention.

A food grade or pharmaceutically acceptable carrier therefor may additionally be used in the manufacture of the composition.

The use of sterol/stanol/ascorbic acid derivatives and salts thereof in the field of weight control and management has not heretofore been investigated or the advantages appreciated. It has been found, within the scope of the present invention, that the derivatives described herein have a surprising and quite unexpected effect on modifying weight. While the mechanism of action is unclear, the results are unequivocal.

In addition, the derivatives of the present invention can be prepared and used as such or they can be easily incorporated into foods, beverages, pharmaceuticals and nutraceuticals regardless of whether these "vehicles" are water-based due to the nature of the derivatives. This enhanced solubility generally translates into lower administration dosages of the derivatives in order to achieve the desired dietary or therapeutic effect.

### BRIEF DESCRIPTION OF THE DRAWINGS

The present invention is illustrated by way the following drawings in which:

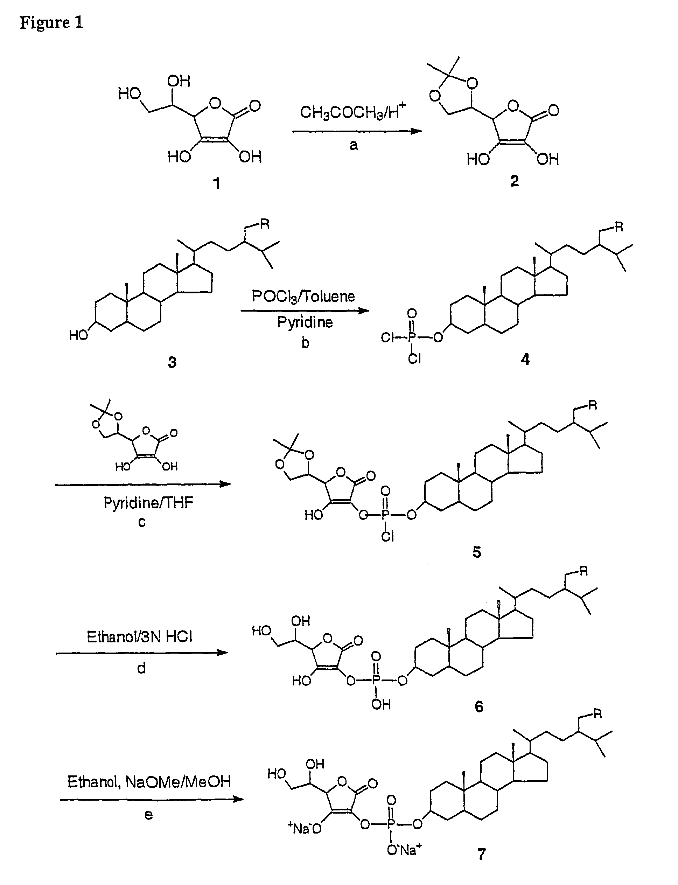

Figure 1 is a schematic showing a process of preparing phytostanol-phosphate-ascorbate and its sodium salt;

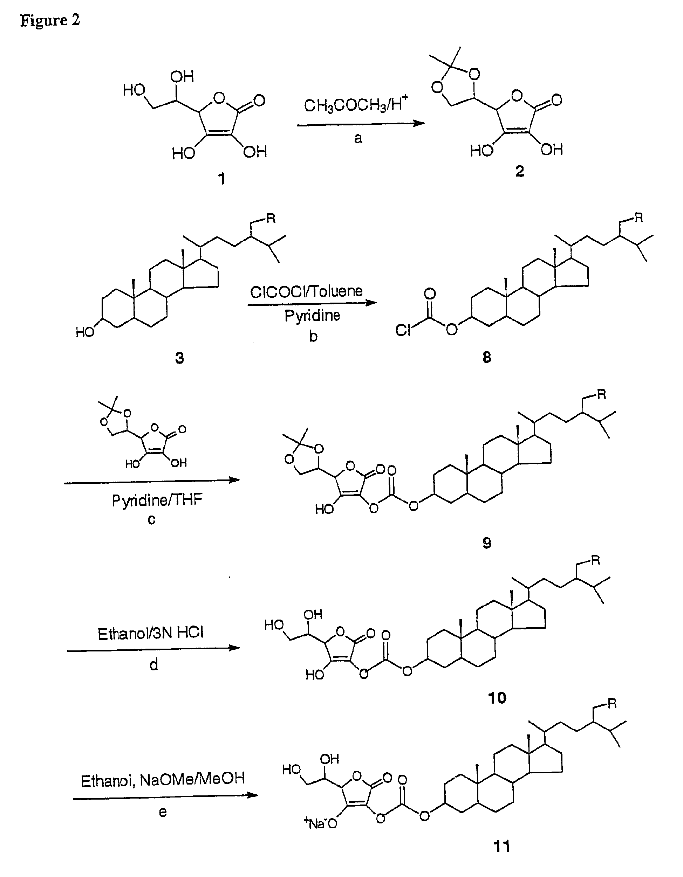

Figure 2 is a schematic showing a process of preparing phytostanol-carbonate-ascorbate and its sodium salt;

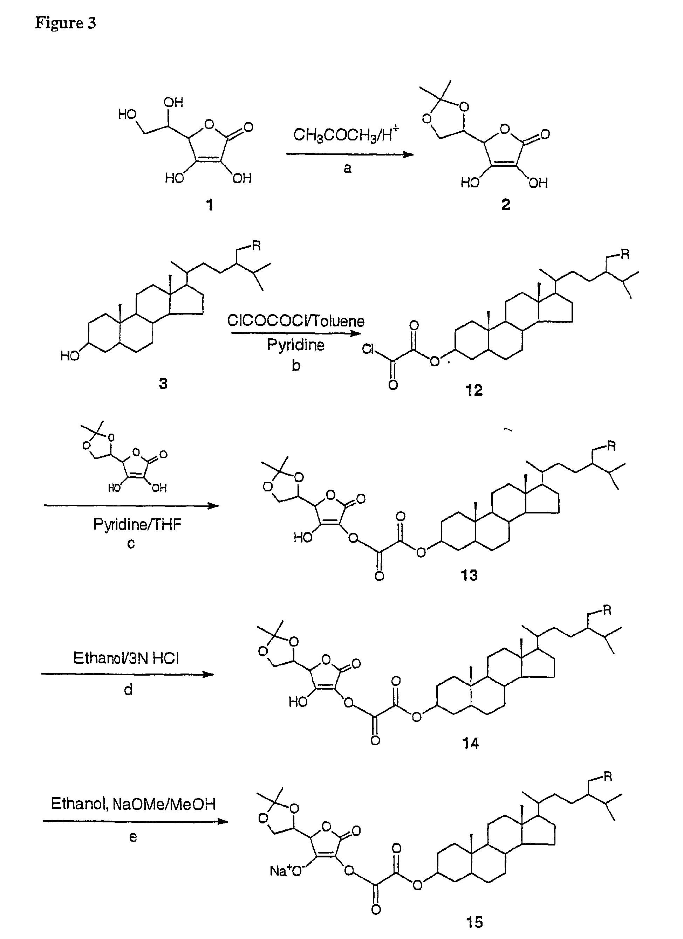

Figure 3 is a schematic showing a process of preparing phytostanol-oxalate-ascorbate and its sodium salt;

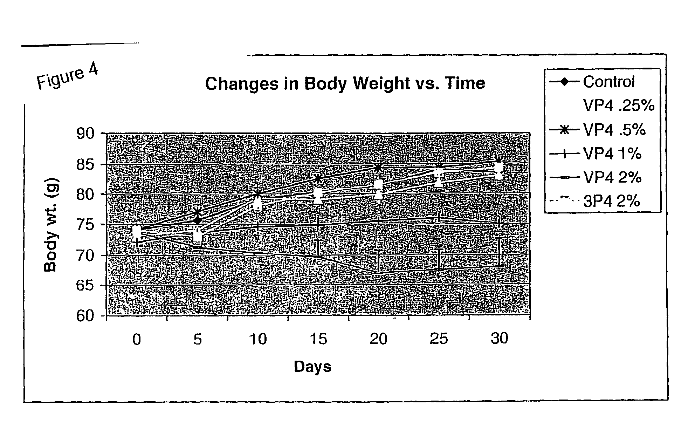

Figure 4 is a graph showing the effects of total gerbil body weight following for 4 continuous weeks of dally gerbil chow administration which either contained no VP4 (controls; n=6), 0.2% VP4 (n=6), 0.5% VP4 (n=6), 1.0% VP4 (n=6), 2.0% VP4 (n=6) or 2.0% 3P4 (n=6). Data are shown as means +/- standard deviations. *p<0.05 vs. controls;

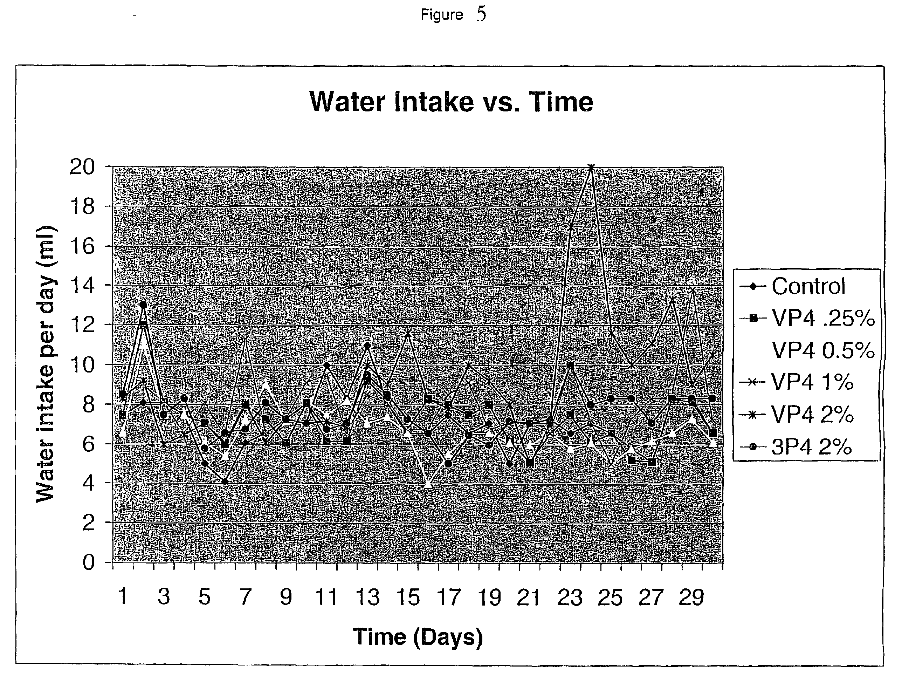

Figure 5 is a graph showing the daily water intake following for 4 continuous weeks of daily gerbil chow administration which either contained no VP4 (controls; n=6), 0.2% VP4 (n=6), 0.5% VP4 (n=6), 1.0% VP4 (n=6), 2.0% VP4 (n=6) or 2.0% 3P4 (n=6). Data are shown as means +/- standard deviations. *p<0.05 vs. controls;

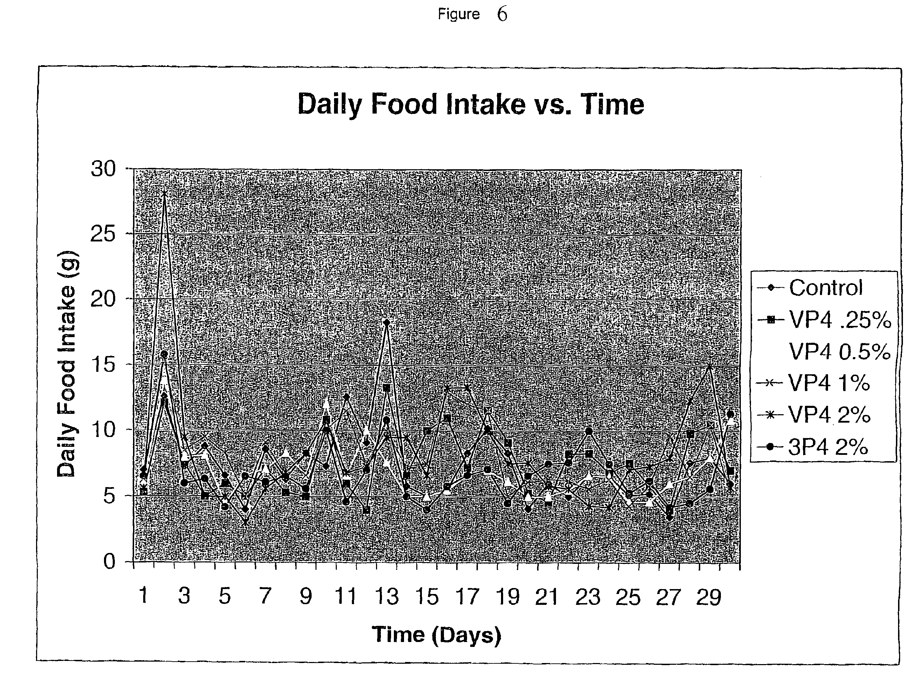

Figure 6 is a graph showing the daily food intake following for 4 continuous weeks of daily gerbil chow administration which either contained no VP4 (controls; n=6), 0.2% VP4 (n=6), 0.5% VP4 (n=6), 1.0% VP4 (n=6), 2.0% VP4 (n=6) or 2.0% 3P4 (n=6). Data are shown as means +/- standard deviations. *p<0.05 vs. controls;

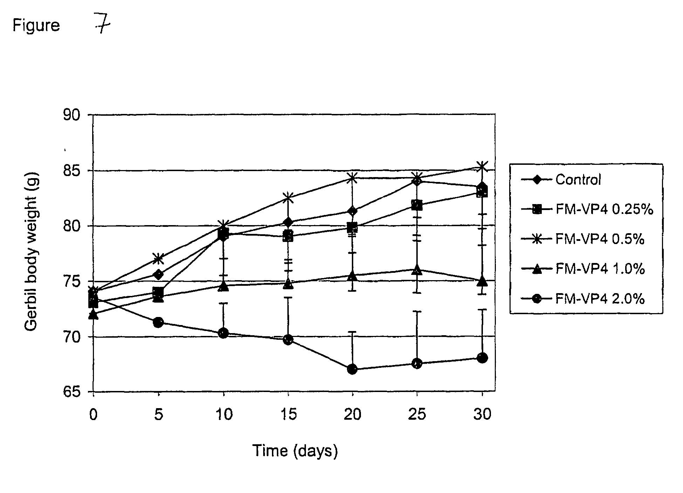

Figure 7 is a graph showing the effect of FM-VP4 on total gerbil body weight following 4 continuous weeks of daily gerbil chow administration containing no FM-VP4 (control) or 0.25, 0.50, 1.0 or 2.0% FM-VP4, with n=6 for each group. Data are shown as mean ± standard deviation. *p<0.05 vs. controls;

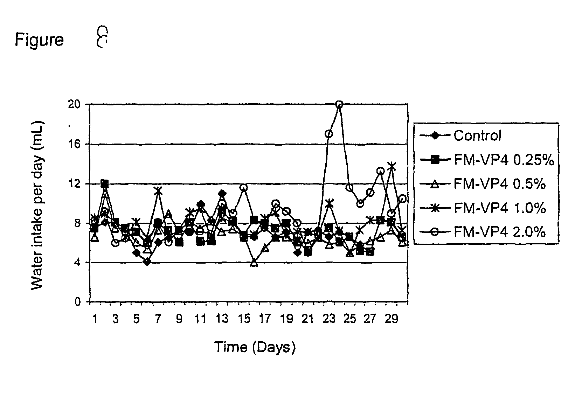

Figure 8 is a graph showing the effect of FM-VP4 on daily water intake in gerbils following 4 continuous weeks of daily gerbil chow administration containing no FM-VP4 (control) or 0.25, 0.50, 1.0 or 2.0% FM-VP4, with n=6 for each group. Data are shown as mean ± standard deviation. *p<0.05 vs. controls; and

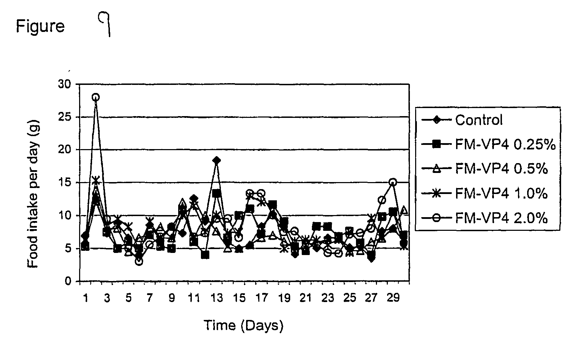

Figure 9 is a graph showing the effect of FM-VP4 on daily food intake in gerbils following 4 continuous weeks of daily gerbil chow administration containing no FM-VP4 (control) or 0.25, 0.50, 1.0 or 2.0% FM-VP4, with n=6 for each group. Data are shown as mean ± standard deviation. *p<0.05 vs. controls;

### PREFERRED EMBODIMENTS OF THE INVENTION

The following detailed description is provided to aid those skilled in the art in practising the present invention.

According to the present invention, there are provided for the use of derivatives of sterol and/or stanol and ascorbic acid in the manufacture of a composition for decreasing weight gain and/or increasing weight loss in animals, including humans. The derivatives of the present invention are represented by one of the following formulae: 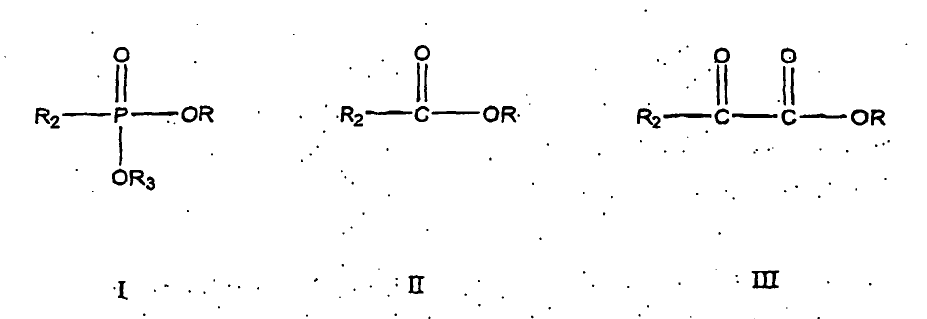 wherein R is a sterol or stanol moiety, R2 is an ascorbic acid moiety and R3 is hydrogen or any metal, alkali earth metal, or alkali metal. The components of the derivative will be described in more detail below. It should be noted that, throughout this disclosure, the terms "derivative", "structure" and "analogue" are used interchangeably to describe the unitary compound which links both a sterol or stanol and ascorbic acid. The elements of the compounds will be described in more detail below

### Sterols/Stanols

As used herein, the term "sterol" includes all sterols without limitation, for example: sitosterol, campesterol, stigmasterol, brassicasterol, desmosterol, chalinosterol, poriferasterol, clionasterol, ergosterol, coprosterol, codisterol, isofucosterol, fucosterol, clerosterol, nervisterol, lathosterol, stellasterol, spinasterol, chondrillasterol, peposterol, avenasterol, isoavenasterol, fecosterol, pollinastasterol, cholesterol, including isomers. The term "stanol" includes all saturated or hydrogenated sterols; including isomers. It is to be understood that modifications to the sterols and stanols i.e. to include side chains also falls within the purview of this invention. It is also to be understood that, when in doubt throughout the specification, the term "sterol" encompasses both sterol and stanol i.e. the terms may be used interchangeably unless otherwise specified.

The sterols and stanols for use in forming derivatives in accordance with this invention may be procured from a variety of natural sources. For example, they may be obtained from the processing of plant oils (including aquatic plants) such as corn oil and other vegetable oils, Wheat germ oil, soy extract, rice extract, rice bran, rapeseed oil, sunflower oil, sesame oil and fish (and other marine-source) oils. The present invention is not to be limited to any one source of phytsterols. US Patent Serial No. 4,420,427 teaches the preparation of sterols from vegetable oil sludge using solvents such as methanol, Alternatively, phytosterols and phytostanols may be obtained from tall oil pitch or soap, by-products of forestry practises as described in US Patent Serial No.5,770,749.

In one preferred form, the derivative of the present invention is formed with naturally-derived or synthesized beta-sitosterol, campestanol, sitostanol and campesterol and each of these derivatives so formed may then be admixed a composition prior to delivery in various ratios. In another preferred form, the derivative of the present invention is formed with naturally-derived or synthesized sitostanol or with naturally derived or synthesized campestanol or mixtures thereof.

### R2

R2 is an ascorbic acid moiety. What is achieved within the scope of the present invention is the creation of a new structure or compound wherein a sterol or stanol moiety is chemically linked to ascorbic acid. The union benefits and enhances the both parts of this new structure. The sterol moiety, formerly poorly soluble, becomes, as part of the new derivative, much more readily soluble in aqueous and non-aqueous media such as oils and fats. Accordingly, administration of the sterol becomes possible without any further enhancements to modify its delivery.

For many years, it has been recognized that L-ascorbic acid (commonly known as vitamin C) is a vital part of balanced human nutrition and plays a role as a physiological antioxidant. However, ascorbic acid is the least stable vitamin with which to work since it reacts extremely easily with atmospheric oxygen yielding dehydroascorbic acid which further and readily decomposes into compounds void of vitamin C efficacy. It is believed that the new structure of the present invention "protects" ascorbic acid from such decomposition. Furthermore, it is believed that the weight loss function of the derivatives of the present invention may be due to the properties of the unique compounds which are formed herein. Linking ascorbic acid with sterols or stanols for the purpose of preparing a weight controlling agent has never been appreciated or explored.

### R3

R3 may be hydrogen or may convert the parent compound into a salt. The over-riding consideration in the selection of the appropriate salt is that they are acceptable pharmaceutically, nutraceutically or for use in foods, beverages and the like. Such salts must have an acceptable anion or cation. Within the scope of the present invention, suitable acid addition salts include those derived from inorganic acids such as hydrochloric, hydrobromic, phosphoric, metaphosphorice, nitric, sulfonic and sulfuric acids and organic acids such as acetic, benzenesulfonic, benzoic, citric, ethanesulfonic, fumaric, gluconic, glyconic, glycolic, isothionic, lactic, lactobionic, maleic, malic, methanesulfonic, succinic, toluenesulfonic, and tartaric.

Suitable base salts include ammonium salts, or any salt of a metal, alkali earth metal or alkali metal. Preferably, R3 is selected from one of: calcium, magnesium, manganese, copper, zinc, sodium, potassium and lithium. Most preferably, R3 is sodium.

In a most preferred form of the present invention, the compound is structure 1 noted above, the stanol is sitostanol or campestanol and R3 is hydrogen or sodium.

### Derivative Formation

### a) Ester Formation

There are many processes by which novel structures comprising sterols and/or stanols and ascorbic acid can be formed. In general, the selected sterol or stanol (or halophosphate, halocarbonate or halo-oxalate derivatives thereof) and ascorbic acid are mixed together under reaction conditions to permit condensation of the "acid" moiety with the "alcohol" (phytosterol). These conditions are the same as those used in other common esterification reactions such as the Fisher esterification process in which the acid component and the alcohol component are allowed to react directly or in the presence of a suitable acid catalyst such as mineral acid, sulfuric acid, phosphoric acid, p-toluenesulfonic acid. The organic solvents generally employed in such esterification reactions are ethers such as diethyl ether, tetrahydrofuran, or benzene, toluene or similar aromatic solvents and the temperatures can vary from room to elevated temperatures depending on the reactivity of the reactants undergoing the reaction.

In a preferred embodiment, the process to form the ester derivative comprises "protecting" the hydroxyl groups of the ascorbic acid or derivatives thereof as esters (for example, as acetate esters) or ethers (for example, methyl ethers) and then condensing the protected ascorbic acid with the sterol/phytostanol halophospahte, halocarbonate or dhalo-oxalate under suitable reaction conditions. In general, such condensation reactions are conducted in an organic solvent such as diethyl ether, tetrahydrofuran, or benzene, toluene or similar aromatic solvents. Depending on the nature and reactivity of the reactants, the reaction temperatures may vary from low (-15°C) to elevated temperatures.

Figure 1 is a schematic showing the formation of the "protected" ascorbic acid (step a), the formation of the intermediary chlorophosphate/stanol derivative (step b), and the condensation reaction (alternatively steps c or d) yielding one of novel derivatives of the present invention based on formula I: phytostanol-phosphate-ascorbate (noted as structure 6).

In more detail, the process shown in Figure 1 is as follows: ascorbic acid is initially protected from decomposition by the formation of 5,6-isopropylidene-ascorbic acid (structure 2). This can be achieved by mixing acetone with ascorbic acid and an acidic catalyst such as sulfuric acid or hydrochloric acid under suitable reaction conditions (refer to Example 1 below). Phytostanol chlorophosphate (structure 4) is prepared by forming a solution of phytostanol in toluene and pyridine (although other nitrogen bases such as aliphatic and aromatic amines may alternatively be used) and treating this solution with a phosphorus derivative such as phosphorus oxychloride. The residue so formed after filtration and concentration of the mother liquor is phytostanol chlorophosphate (structure 4). The latter is then mixed with 5,6-isopropylidene-ascorbic acid and, after the addition of a suitable alcohol such as ethanol and HCl (step d), concentrated. Alternatively, pyridine/THF may be added (step c) and the product concentrated. After final washing and drying (step e), the resultant novel product of both steps c or d is phytostanol-phosphate-ascorbate (structure 6).

In another preferred form of the process of the present invention, ascorbic acid is protected at the hydroxyl sites not as 5,6-isopropylidene-ascorbic acid but as esters (for example as acetates, phosphates and the like.). The latter may then be condensed with phytosterols or phytostanols, derivatized as described above, using known esterification methods ultimately to produce the structures of the present invention. The formation of mono and diphosphates of ascorbic acid is described thoroughly in the literature. For example, US Patent Serial No. 4,939,128 to Kato et al., teaches the formation of phosphoric acid esters of ascorbic acid. Similarly, US Patent Serial No. 4,999,437 to Dobler et al., describes the preparation of ascorbic acid 2-phosphate. In Dobler et al., the core reaction of phosphorylating ascorbic acid or ascorbic acid derivatives with POCl3 in the presence of tertiary amines (described in German Laid Open Application DOS 2,719,303) is improved by adding to the reaction solution a magnesium compound, preferably an aqueous solution of a magnesium compound. Any of these known ascorbic acid derivatives can be used within the scope of the present invention.

Figure 2 is a schematic showing the formation of the "protected" ascorbic acid (step a), the formation of the intermediary chlorocarbonate/stanol derivative (step b), and the condensation reaction (optionally steps c or d) yielding structure 9 (10 is the same), one of novel derivatives of the present invention based on formula II: phytostanol-carbonate-ascorbate. These chlorocarbonate derivatives may be prepared by the same process outlined in detail above with respect to Figure 1; however, the phosphorus oxylchloride is replaced (as shown in step b of Figure 2) by phosgene.

Figure 3 is a schematic showing the formation of the "protected" ascorbic acid (step a), the formation of the intermediary chloro-oxalate/stanol derivative (step b), and the condensation reaction (optionally steps c or d) yielding a novel structure 13 (same as 14), one of novel derivatives of the present invention based on formula III: phytostanol-oxalate-ascorbate (noted as structure 14). These chloro-oxalate derivatives may be prepared by the same process outlined in detail above with respect to Figure 1; however, the phosphorus oxylchloride is replaced (as shown in step b of Figure 3) by oxalyl chloride.

### b) Salt Formation

The present invention encompasses not only the parent structures comprising sterols or phytostanols and ascorbic acid (for example, those preferred structures shown as structures 5 and 6 in Figure 1, structures 9 an 10 in Figure 2 and structures 13 and 14 in Figure 3) but also the salts thereof. These salts are even more water-soluble than the corresponding parent compounds and therefore their efficacy and evaluation both *in vitro* and *in vivo* are much improved.

Salt formation of the derivatives of the present invention can be readily performed by treatment of the parent compound with a series of bases (for example, sodium methoxide or other metal alkoxides) to produce the corresponding alkali metal salts. Other metal salts of calcium, magnesium, manganese, copper, zinc, and the like can be generated by reacting the parent with suitable metal alkoxides. With respect to formula I, R3 represents either hydrogen (parent compound) or any metal, alkali earth metal, or alkali metal (the salt).

### c) Reduction by Catalytic (Hydrogenation) and Chemical Methods

Optionally, the sterol derivatives of the present invention or the constituent moieties thereof (either the sterol or the ascorbic acid) prior to or after derivative formation may be hydrogenated or saturated. The hydrogenation of heterocyclic ring systems to the partially or fully reduced analogues is a well known process. For example, the catalytic and/or chemical reduction of the ring of ascorbic acid to the corresponding dihydro analogue is readily accomplished under an atmosphere of hydrogen and a metal catalyst such as platinum, palladium or Raney Nickel. In general, this reduction is performed in an organic solvent such as ethanol, ethyl acetate or similar media and either under atmospheric pressure or at a low pressure (3-5 psi) at room temperature or slightly elevated temperatures.

The chemical reductions of such systems involve reduction with a family of "hydride" reagents such as sodium borohydride, lithium aluminum hydride and their analogues. These reductions are generally performed in an anhydrous inert medium involving ethyl ether, tetrahydrofuran, dioxane, or benzene, toluene or similar aromatic solvents at room to reflux temperatures.

Similar catalytic or chemical processes can be applied to all of the phytosterol analogues of the present invention. Accordingly, the present invention includes within its scope a ascorbic acid moiety and a phytosterol moiety.

### Derivatives

The present invention comprises all derivatives of sterol and/or stanol and ascorbic acid, including salts thereof represented by the general formulae: 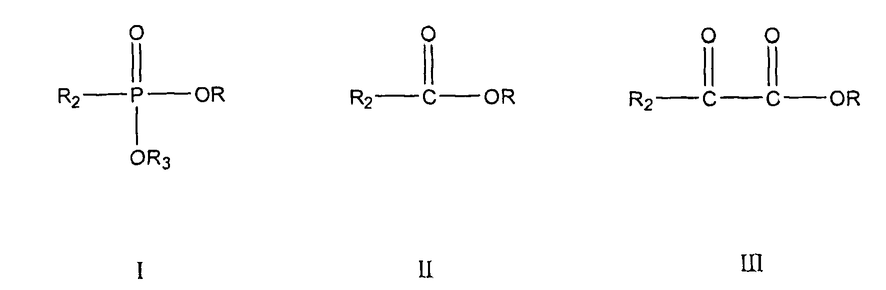 wherein R is a sterol or stanol moiety; R2 is derived from ascorbic acid and R3 is hydrogen or any metal, alkali earth metal, or alkali metal. Most preferably, the present invention comprises all halophosphate, halocarbonate and halo-oxalate/phytostanol/ascorbate derivatives as shown in Figures 1 through 3 as structures 5, 6, 7, 9, 10, 11 13 14 and 15. It is to be clearly understood; however, that these structures are only a selection of the many novel derivatives which fall within the purview of formulae I, II and III. It is also to be understood that although sodium salts are shown in structures 7, 11 and 15, other salts are included within the scope of the invention, as described above.

### Advantages of Sterol/Stanol Analogues

The derivatives of the present invention, wherein ascorbic acid is attached to the sterol moiety affords many dietary and therapeutic advantages. What has been found, within the scope of the present invention, is that these derivatives are particularly useful in regulating weight, more specifically, decreasing weight gain and increasing weight loss as required.

In addition, these derivatives are easy to use from a manufacturing and commercial perspective. They are readily soluble, both in aqueous solutions and non-aqueous media such as oils and fats.

### Delivery Systems

Although it is fully contemplated within the scope of the present invention that the derivatives may be administered to animals, particularly humans, directly and without any further modification, it is possible to take further steps to enhance delivery and ensure even distribution throughout the food, beverage, pharmaceutical, nutraceutical and the like to which they are added. It is to be understood; however, that these steps are purely optional. Such enhancement may be achieved by a number of suitable means such as, for example, solubilizing or dispersing the derivatives to form emulsions, solutions and dispersions or self-emulsifying systems; lyophilizing, spray drying, controlled precipitating, or a combination thereof; forming solid dispersions, suspensions, hydrated lipid systems; forming inclusion complexations with cyclodextrins; and using hydrotopes and formulations with bile acids and their derivatives.

Each of the techniques, which may be used in delivery, are described in more detail in PCT/CA99/00512.

### Methods of Use

The derivatives of the present invention may be administered to animals, in particular humans, directly and without further modification or may be treated to enhance further the solubility and/or dispersability of the composition as described in detail above. Alternatively, and optionally in conjunction with any one of these solubility and/or dispersability enhancement methods, the derivatives may be incorporated into various vehicles as described further below in order to modulate or control body weight. For example, these derivatives may be combined, in therapeutically effective amounts, with customary carriers and excipients in the fields of pharmaceuticals, nutraceuticals or functional foods and administered as required. In populations, which are considered "high-risk" for weight-related disorders or obesity, it is contemplated that the derivatives of the present invention be used in primary, secondary and tertiary treatment programs.

Accordingly without limiting the generality of the foregoing, the derivatives of the present invention may be admixed with various carriers or adjuvants to assist in direct administration or to assist in the incorporation of the composition into foods, beverages, nutraceuticals or pharmaceuticals. In order to appreciate the various possible vehicles of the delivery of the derivatives, the list below is provided. The doses of the derivatives will vary depending upon, among other factors, the mode of delivery, the patient size and condition, the result to be achieved, as well as other factors known to those skilled in the art of food additives and medicinal agents. Naturally, an amount of the compound(s) should be administered to achieve the desired therapeutic or prophylactic result i.e. to achieve the goals of:
a) preventing weight gain in an individual; and/or
b) weight loss in an individual.

### 1) Pharmaceutical Dosage Forms:

It is contemplated within the scope of the present invention that the derivatives of the present invention may be incorporated into various conventional pharmaceutical preparations and dosage forms such as tablets (plain and coated) for use orally, bucally or lingually, capsules (hard and soft, gelatin, with or without additional coatings) powders, granules (including effervescent granules), pellets, microparticulates, solutions (such as micellar, syrups, elixirs and drops), lozenges, pastilles, ampoules, emulsions, microemulsions, ointments, creams, suppositories, gels, transdermal patches and modified release dosage forms together with customary excipients and/or diluents and stabilizers.

The derivatives of the present invention, adapted into the appropriate dosage form as described above may be administered to animals, including humans, orally, by injection (intravenously, subcutaneously, intra-peritoneally, intra-dermally or intra-muscularly), topically or in other ways.

### 2) Foods/Beverages/Nutraceuticals:

In another form of the present invention, the derivatives of the present invention may be incorporated into foods, beverages and nutraceuticals, including, without limitation, the following:
1) Dairy Products --such as cheeses, butter, milk and other dairy beverages, spreads and dairy mixes, ice cream and yoghurt;
2) Fat-Based Products--such as margarines, spreads, mayonnaise, shortenings, cooking and frying oils and dressings;
3) Cereal-Based Products--comprising grains (for example, bread and pastas) whether these goods are cooked, baked or otherwise processed;
4) Confectioneries--such as chocolate, candies, chewing gum, desserts, non-dairy toppings (for example Cool Whip^{™}), sorbets, icings and other fillings;
5) Beverages-- whether alcoholic or non-alcoholic and including colas and other soft drinks, juice drinks, dietary supplement and meal replacement drinks such as those sold under the trade-marks Boost^{™} and Ensure^{™}; and
6) Miscellaneous Products--including eggs and egg products, processed foods such as soups, pre-prepared pasta sauces, pre-formed meals and the like.

The derivatives of the present invention may be incorporated directly and without further modification into the food, nutraceutical or beverage by techniques such as mixing, infusion, injection, blending, dispersing, emulsifying, immersion, spraying and kneading. Alternatively, the derivatives may be applied directly onto a food or into a beverage by the consumer prior to ingestion. These are simple and economical modes of delivery.

### EXAMPLES

The present invention is described by the following examples:

### Example 1

### Protection of ascorbic Acid

Oleum (24%, 8.3g) was added dropwise to acetone (50ml). Ascorbic acid (12g) was introduced to the mixture at 0°C and the reaction mixture was stirred at 0°C for 6 hours. The obtained crystals were filtered off under suction, the filtered cake was pressed to dryness and then washed with acetone (30ml). The product, 5,6-isopropylidene ascorbic acid (14g) was obtained.

### Example 2

### Attachment to Phytostanols

A solution of phytostanol mixture (24g) (campestanol: 36.4%; sitostanol: 62.3%) in toluene (500ml) and pyridine (25ml) was added dropwise to a mixture of phosphorous oxychloride (9ml) in toluene (200ml) at 0°C. The mixture was stirred at room temperature for 3 hours. The pyridine hydrochloride was filtered off and the mother liquor was concentrated to recover the toluene. The residue was dissolved in dry THF (100ml) and a solution of the above-prepared protected ascorbic acid (14g) in dry THF (400ml) was added dropwise at 0°C. The stirring at room temperature was maintained for 1 hr. The solution was concentrated to remove the solvent. Ethanol (400ml) and 3N HCl (200ml) were added, the mixture was heated to 50°C for 30 min and concentrated. Ethyl acetate (600ml) was added, the resultant solution was washed with water (3X300ml), dried over sodium sulfate, concentrated and the product (phytostanol-phosphate-ascorbate) was obtained as a white powder 22g.

### Example 3

### Conversion to Sodium Salt

The above-prepared acid (17g) was dissolved in ethanol (100ml) and a solution of sodium methoxide (2.7g) in ethanol (50ml) was added at stirring and at room temperature. The stirring was maintained for 30 min. after the addition. The resultant white cake was filtered off, dried and weighed, to afford a white powder 20g (phytostanol-phosphate-ascorbate sodium).

### Example 4

Effects of one of the compounds of the present invention, phytostanol-phosphoryl-ascorbate, herein referred to interchangeably as "FCP-VP4" or "FM-VP4" (at various doses) and FCP-3P4 (positive control) on weight following chronic oral administration to gerbils.

Gerbils were administered a standard gerbil diet for 4 continuous weeks (daily water and food intake was monitored and replaced) which consisted of either no VP4 (controls; n=6), 0.25% wt/wt, VP4 (n=6), 0.5% wt/wt VP4 (n=6), 1.0% wt/wt VP4 (n=6), 2.0% wt/wt VP4 (n=6) or 2.0% wt/wt 3P4 incorporated into the gerbil chow (n=6). Following 4 weeks of receiving one of these treatments blood was obtained *via* a cardiac puncture and all animals were sacrificed humanely.

Animals administered 1% or 2% FCP-VP4 had significantly lower body weight following the 4 weeks of treatment compared to the other groups. However, no visible signs of distress were observed in these animals.

Different concentrations of FCP-VP4 (0.25, 0.5, 1, 2% wt/wt) and FCP-3P4 (2% wt/wt) were incorporated into standard gerbil chow by standard milling procedures (1). Liquid chromatography-mass spectrometry analysis was used to confirm the percentage of FCP-3P4 or -VP4 incorporated into the gerbil chow (data not shown). All gerbils used in this study were cared for in accordance with the principles promulgated by the Canadian Council on Animal Care and the University of British Columbia. Adult male Mongolian gerbils (70-80 g) were obtained from Charles River Breeders (Montreal, Quebec, Canada). All treatment groups were maintained under a 12 h light (0700-0900)/dark cycle and supplied with a standard laboratory diet (PMI Feeds, Richmond, VA, USA) and water *ad libitum.*

Gerbils were provided a standard gerbil diet for 4 continuous weeks (daily water and food intake was monitored and replaced) which consisted of either no FCP-VP4 (controls: n=6), 0.25% FCP-VP4 (n=6), 0.5% FCP-VP4 (n=6), 1.0% FCP-VP4 (n=6), 2.0% FCP-VP4 (n=6) or 2.0% FCP-3P4 (n=6). Following 4 weeks of this regiment blood was obtained *via* a cardiac puncture and all animals were sacrificed humanely

Differences in body weight, daily food and water intake of all treatment and control groups were determined using an analysis of variance (PCANOVA; Human Dynamic Systems). Critical differences were determined by post-hoc Newman Keuls test. Differences were considered significant if P < 0.05. All data are expressed as mean +/standard deviation.

Animals administered 1% or 2% FCP-VP4 had significantly lower body weight following the 4 weeks of treatment compared to the other groups (Figure 4). However, no visible signs of distress were observed in these animals. No significant differences in daily water (Figure 5) or food intake (Figure 6) were observed in all treatment groups compared to controls.

### Example 5

### 28 day Study on Beagle Dogs

Twenty-four pure-bred beagle dogs (12 males and 12 females) were divided into 4 groups, each consisting of 3 males and 3 females. Three groups received the preferred compound of the present invention, referred to as FM-VP4, administered in gelatin capsules at doses of 50, 250 or 800 mg/kg/day for 4 weeks. The dose was given each day as two equal subdoses with an interval of approximately 6 hours. The other group received empty gelatin capsules twice each day and acted as controls.

The following parameters were evaluated during the study; clinical signs, bodyweight, food consumption, ophthalmic examination (before dosing commenced and Week 4), haematology, blood chemistry (before dosing commenced and during Weeks 2 and 4), urinalysis (before dosing commenced and Week 4), electrocardiography and blood pressure measurements (before dosing commenced and during Week 4), organ weights, macroscopic pathology and histopathological examination of the tissues.

The following principal findings are given in summary:

### Mortality

There were no unscheduled deaths.

### Bodyweight and food consumption

There was a significant effect on bodyweight for both sexes receiving 800 mg/kg/day and females receiving 250 mg/kg/day.

### Ophthalmic examination

There was no effect of treatment.

### Haematology

There were no findings related to treatment.

### Alanine aminotransferase

(ALT) was increased during Weeks 2 and 4 for some or all males receiving 800 mg/kg/day.

### Example 6 Oral Administration to Beagle Dogs for One Week Study

Two pure-bred beagle dogs (1 male and 1 female) were dosed with the preferred compound of the present invention by oral capsule twice daily for 7 days. The scheduled total daily dose of 2000 mg/kg/day was administered for 3 days.

Both animals were weighed, prior to feeding, once a week throughout the pre-dosing period. In addition, they were weighed on days 1, 4 and 8.

Both the male and female dogs showed weight loss during the dosing period of 0.3 and 0.8 kg respectively.

**Table 1: Individual Body weight Data (kg)**

| Day | Male Dog | Female Dog |
|---|---|---|
| -13 | 12.7 | 10.3 |
| -6 | 12.5 | 10.8 |
| 1 | 12.7 | 10.8 |
| 4 | 12.8 | 10.7 |
| 8 | 12.4 | 10.0 |

### Example 7 Juvenile Gerbil Study Over Four Weeks

Standard milling procedures (1) were used to incorporate different concentrations of the preferred compound of the present invention, FM-VP4 (0.25, 0.5, 1, 2% w/w) into standard gerbil chow. Mongolian gerbils were used in these studies and were cared for in accordance with the Canadian Council on Animal Care and the University of British Columbia guidelines. Thirty adult male Mongolian gerbils (70-80 g) were obtained from Charles River Breeders (Montreal, Canada). The gerbils were maintained under a 12 hr light (0700-1900)/dark cycle and supplied with a standard laboratory gerbil diet (Jameison's Pet Food Distributor, Delta, B.C.) and water *ad libitum.* After a 2-week adaptation period, the gerbils were divided into fives groups of six animals matched for body weight and administered a standard gerbil diet containing either no FM-VP4 (control) or FM-VP4 at 0.25, 0.5, 1, 2% w/w for 4 continuous weeks. Food and water was supplied *ad libitum* and daily intake was monitored and replaced.

### Statistical Analysis

Differences in body weight for all treated and control gerbils were determined using an analysis of variance (Instat2; GraphPad). Significant differences were determined using a Newman Keuls post-hoc test where differences were considered significant if p<0.05. All data are expressed as mean ± standard deviation.

### Results and Discussion

Animals administered 1% or 2% (w/w) dietary FM-VP4 had significantly lower body weight following 4 weeks of treatment compared to the other groups (Figure 7). No visible signs of distress or illness were observed in these animals. Control gerbils, given no FM-VP4, exhibited a significant weight increase (13%, p<0.001) at 30 days compared to baseline (Figure 7). Gerbils fed FM-VP4 at 1% (w/w) exhibited minimal weight gain and had a significantly lower body weight (-11%, p<0.01) than the control animals after thirty days. Gerbils fed 2% FM-VP4 also had significantly lower body weight (-23%, p<0.001) compared to control animals and experienced a slight but not significant reduction in weight (-8%) compared to baseline.

For the most part no significant differences in daily water (Figure 8) or food intake (Figure 9) were observed during the study. However, daily water intake in the gerbils receiving 2% FM-VP4 was significantly greater than the other treatment groups from days 23 to 30. The prevention of weight gain in the 1 and 2% FM-VP4 groups (Figure 7) was likely not due to decreased food or water intake, as there was no trend toward decreased food or water consumption for these groups (Figures 8 and 9).
Conclusion: The results show that FM-VP4 is effective in the prevention of weight gain.

### Example 7 Adult Gerbil Study Over Eight Weeks

Standard milling procedures (1) were used to incorporate different concentrations of the preferred compound of the present invention, FM-VP4 at 2% & 4% w/w into standard gerbil chow. FM-VP4 (2% & 4% w/v) was also dissolved into drinking water. Fresh drinking water and gerbil chow was replaced on a daily basis throughout the duration of the study.

The experimental protocol was similar to that used in example 6. Thirty adult male Mongolian gerbils (70-80 g) were obtained from Charles River Breeders (Montreal, Canada). The gerbils were maintained under a 12 hr light (0700-1900)/dark cycle and supplied with a standard laboratory gerbil diet (Jameison's Pet Food Distributor, Delta, B.C.) and water *ad libitum.* After a 2-week adaptation period, the gerbils were divided into six treatment groups matched for body weight and the following treatment protocol was used throughout the duration of the study: Gerbils had access to water and food daily *ad litium* for 8 continuous weeks (daily water and food intake were monitored and replaced), which contains either no FM-VP4 (controls; n=6), 2.0% FM- VP4 incorporated into the gerbil diet or dissolved into the drinking water (n=6) and 4.0% FM-VP4 incorporated into the gerbil diet (n=6) or dissolved into the drinking water (n=6).

Differences in daily food and water intake, AST, ALT and creatinine levels and body weight for all treated and control gerbils were determined using an analysis of variance (Instat2; GraphPad). Significant differences were determined using a Newman Keuls post-hoc test where differences were considered significant if p<0.05. All data are expressed as mean ± standard error of the mean.

Animals administered 4% FM-VP4 in either their diet or drinking water had significantly lower body weight following the 8 weeks of treatment compared to the other groups (Table 2). Significant differences in daily water intake was observed in all treatment groups with the exception of the 2% FM-VP4 in diet group compared to controls (Table 3). Significant differences in daily food intake were observed in gerbils administered 2% FM-VP4 in the drinking water and 4% FM-VP4 in the diet and drinking water groups compared to control (Table 4). A significant decrease in plasma AST, ALT and creatinine concentrations were not observed in all treatment groups compared to controls (Table 5). Taken together these findings suggest that 2% FM-VP4 incorporated into the gerbil food or drinking water significantly decrease body weight with no visible signs of animal distress or hepatic and renal toxicity compared to controls.

### REFERENCES

1. S. Guinot and F. Leveiller. The use of MTDSC to assess the amorphous phase content of a micronized drug substance. *Int. J. Pharm.* **192**:63-75 (1999).

**Table 2. Effect of 8-week treatment with FM-VP4 in diet and/or water on gerbil body weight during each week of the study.**

| Treatment Group | Week 0 | Week 1 | Week 2 | Week 3 | Week 4 | Week 5 | Week 6 | Week 7 | Week 8 |
|---|---|---|---|---|---|---|---|---|---|
| (Body Weight; grams/gerbil) | | | | | | | | | |
| **Controls** | 81.5±2.5 | 83.0±2.7 | 86.2±3.0 | 86.8±3.8 | 88.7±4.2 | 89.0±4.6 | 88.5±4.9 | 88.8±5.4 | 90.0±5.8 |
| **FM-VP4 2% within diet** | 83.3±2.2 | 80.6±1.8 | 78.6±1.8^{a} | 77.0±2.2^{a} | 78.0±2.4^{a} | 79.2±2.1^{a} | 79.7±2.4^{a} | 81.0±2.7 | 82.6±2.5 |
| **FM-VP4 4% within diet** | 81.6±1.4 | 74.6±1.4^{a,b} | 68.3±2.1^{a,b} | 64.7±1.8^{a,b} | 64.0±1.8^{a,b} | 63.3±2.0^{a,b} | 64.2±2.5^{a,b} | 66.7±2.4^{a,b} | 67.3±2.5^{a,b} |
| **FM-VP4 2%** in water | 77.6±1.1^{b,c} | 75.0±1.2^{a,b} | 77.3±1.2^{a,c} | 77.0±1.1^{a,c} | 78.5±1.6^{a,c} | 79.3±2.0^{a,c} | 84.0±2.1^{c} | 84.6±2.4^{c} | 85.0±2.8^{c} |
| **FM-VP4 4% in water** | 81.0±2.4 | 69.6±1.8^{a,b,c,d} | 68.0±3.0^{a,b,d} | 67.0±3.6^{a,b,d} | 65.8±4.4^{a,b,d} | 65.5±5.1^{a,b,d} | 67.7±5.4^{a,b,d} | 66.8±5.3^{a,b,d} | 68.0±5.1^{a,b,d} |

| | | | | | | | | | |
|---|---|---|---|---|---|---|---|---|---|
| Data presented as mean ± standard error of the mean (SEM); n=6 for all treatment groups. ^{a}P<0.05 vs. controls; ^{b}P<0.05 vs. FM-VP4 2% in diet; ^{c}P<0.05 vs. FM-VP4 4% in diet; ^{d}P<0.05 vs. FM-VP4 2% in water. | | | | | | | | | |

**Table 3. Effect of 8-week treatment with FM-VP4 in diet and/or water on average gerbil water intake (ml/day/gerbil) during each week of the study.**

| Treatment Group | Week 1 | Week 2 | Week 3 | Week 4 | Week 5 | Week 6 | Week 7 | Week 8 |
|---|---|---|---|---|---|---|---|---|
| (ml/day/gerbil) | | | | | | | | |
| **Controls** | 7.6±0.3 | 8.6±1.2 | 9.1±1.2 | 9.1±0.7 | 8.9±1.7 | 10.1±1.7 | 9.5±1.2 | 9.0±0.7 |
| **FM-VP4 2% within diet** | 5.4±0.6^{a} | 6.2±0.7^{a} | 8.7±0.6 | 8.7±0.9 | 8.4±1.1 | 9.6±0.8 | 8.7±0.8 | 8.8±0.6 |
| **FM-VP4 4% within diet** | 4.3±0.3^{a,b} | 5.6±0.9^{a} | 8.3±0.8 | 9.2±0.6 | 9.4±1.0 | 9.6±0.7 | 8.8±0.7 | 8.2±0.4 |
| **FM-VP4 2% in water** | 5.8±1.0^{a,c} | 5.3±1.0^{a} | 5.2±0.5^{a,b,c} | 5.7±0.8^{a,b,c} | 6.0±1.3^{c} | 6.0±0.6^{a,b,c} | 7.4±0.8^{a} | 6.3±0.5^{a,b,c} |
| **FM-VP4 4% in water** | 5.8±0.8^{a,c} | 6.2±1.9 | 4.1±0.6^{a,b,c} | 5.6±0.6^{a,b,c} | 5.6±1.5^{a,b,c} | 6.9±0.8^{a,b,c} | 7.5±1.4 | 7.2±0.8^{a} |

| | | | | | | | | |
|---|---|---|---|---|---|---|---|---|
| Data presented as mean ± standard error of the mean (SEM); n=6 for all treatment groups. ^{a}P<0.05 vs. controls; ^{b}P<0.05 vs. FM-VP4 2% in diet; ^{c}P<0.05 vs. FM-VP4 4% in diet; ^{d}P<0.05 vs. FM-VP4 2% in water. | | | | | | | | |

**Table 4. Effect of 8-week treatment with FM-VP4 in diet and/or water on average gerbil food intake (grams/day/gerbil) during each week of the study.**

| Treatment Group | Week 1 | Week 2 | Week 3 | Week 4 | Week 5 | Week 6 | Week 7 | Week 8 |
|---|---|---|---|---|---|---|---|---|
| (grams/day/gerbil) | | | | | | | | |
| **Controls** | 8.2±0.9 | 6.2±0.4 | 6.2±0.4 | 7.6±1.0 | 6.3±0.6 | 6.6±0.9 | 5.2±0.5 | 5.0±0.3 |
| **FM-VP4 2% within diet** | 5.8±0.8^{a} | 5.6±0.5 | 5.7±0.8 | 6.7±0.7 | 6.8±0.9 | 5.7±0.7 | 6.4±0.4^{a} | 6.1±0.6^{a} |
| **FM-VP4 4% within diet** | 8.8±1.4^{b} | 5.6±0.9 | 7.0±0.9 | 9.3±1.3^{b} | 8.5±1.0^{a} | 8.3±0.7^{a,b} | 8.0±0.9^{a,b} | 8.5±1.0^{a,b} |
| **FM-VP4 2% in water** | 6.7±1.2 | 7.8±0.9^{a,b,c} | 5.2±0.4^{a,c} | 6.2±0.5^{c} | 5.2±0.6^{b,c} | 6.0±0.6^{c} | 5.5±0.5^{c} | 6.3±0.9^{a,c} |
| **FM-VP4 4% in water** | 6.9±1.5 | 7.7±0.9^{b,c} | 7.9±0.6^{a,b,d} | 7.1±0.3^{c,d} | 5.8±0.5^{c} | 6.9±0.5^{c} | 7.2±0.5^{a,d} | 5.8±0.7^{c} |

| | | | | | | | | |
|---|---|---|---|---|---|---|---|---|
| Data presented as mean ± standard error of the mean (SEM); n=6 for all treatment groups. ^{a}P<0.05 vs. controls; ^{b}P<0.05 vs. FM-VP4 2% in diet; ^{c}P<0.05 vs. FM-VP4 4% in diet; ^{d}P<0.05 vs. FM-VP4 2% in water. | | | | | | | | |

**Table 5. Effect of 8-week treatment with FM-VP4 in diet and/or water on plasma creatinine, aspartate aminotransferase (AST) and alanine aminotransferase (ALT) concentrations in adult male gerbils.**

| Treatment Group | Creatinine* (mg/dL) | AST** (SF units/mL) | ALT** (SF units/mL) |
|---|---|---|---|
| **Controls** | 0.49 ± 0.13 (n=6) | 106.1 ± 27.2 (n=6) | 38.0 ± 5.2 (n=6) |
| **FM-VP4 2% in diet** | 0.36 ± 0.02 (n=4) | 106.9 ± 15.0 (n=4) | 23.3 ± 1.5^{a} (n=5) |
| **FM-VP4 4% in diet** | 0.41 ± 0.02^{b} (n=5) | 157.5 ± 15.5^{a,b} (n=4) | 35.9 ± 2.1^{b} (n=4) |
| **FM-VP4 2% in water** | 0.43 ± 0.06 (n=5) | 74.4 ± 10.8^{a,b,c} (n=4) | 25.8 ± 5.2^{a,c} (n=5) |
| **FM-VP4 4% in water** | 0.51 ± 0.17 (n=5) | 65.9 ± 8.3^{a,b,c,d} (n=3) | 20.4 ± 0.7^{a,c} (n=3) |

| | | | |
|---|---|---|---|
| Data presented as mean ± standard error of the mean (SEM); ^{a}P<0.05 vs. controls; ^{b}P<0.05 vs. FM-VP4 2% in diet; ^{c}P<0.05 vs. FM-VP4 4% in diet; ^{d}P<0.05 vs. FM-VP4 2% in water. Abbreviations: AST, aspartate aminotransferase; ALT, alanine aminotransferase; ND, below the detectable limit of the assay. *Elevation in plasma creatinine concentration compared to non-treated controls indirectly suggests kidney damage, specifically the renal filtration mechanism. **Elevations in plasma AST and/or ALT concentration compared to non-treated controls indirectly suggests liver damage. | | | |

## Claims

1. Use of one or more of the structures having the following formulae: 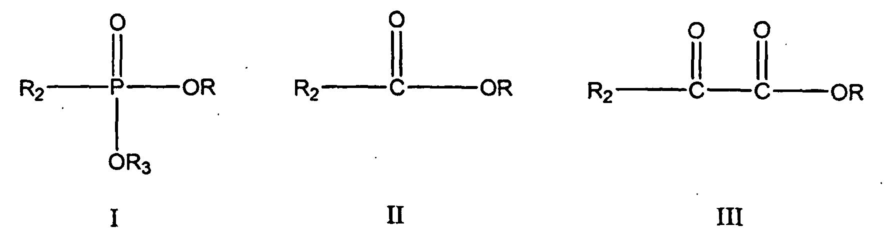 wherein R is a sterol or stanol moiety, R₂ is an ascorbic acid moiety and R₃ is hydrogen or any metal, alkali earth metal or alkali metal; and all salts of these structures in the manufacture of a composition for use in a method of weight control in an animal.

2. Use according to claim 1 wherein the animal is a human.

3. The use of claim 1 wherein the sterol is selected from the group consisting of sitosterol, campesterol, stigmasterol, brassicasterol, desmosterol, chalinosterol, poriferasterol, clionasterol, ergosterol, coprosterol, codisterol, isofucosterol, fucosterol, clerosterol, nervisterol, lathosterol, stellasterol, spinasterol, chondrillasterol, peposterol, avenasterol, isoavenasterol, fecosterol, pollinastasterol and cholesterol.

4. The use of claim 1 wherein the stanol is selected from the group consisting of all saturated or hydrogenated sterols.

5. The use of claim 1 wherein the stanol is sitostanol.

6. The use of claim 1 wherein R₃ is selected from the group consisting of calcium, magnesium, manganese, copper, zinc, sodium, potassium and lithium.

7. The use of any previous claim in which the method of weight control results in a decrease in weight gain.

8. The use of any of claims 1-6 in which the method of weight control results in an increase in weight loss.

9. The use according to any previous claim in which the composition is a comestible or beverage.

10. The use according to any previous claim wherein a food grade or pharmaceutically acceptable carrier therefor is used in the manufacture of the composition.

## Patentansprüche

1. Verwendung einer oder mehrerer der Strukturen mit den folgenden Formeln: 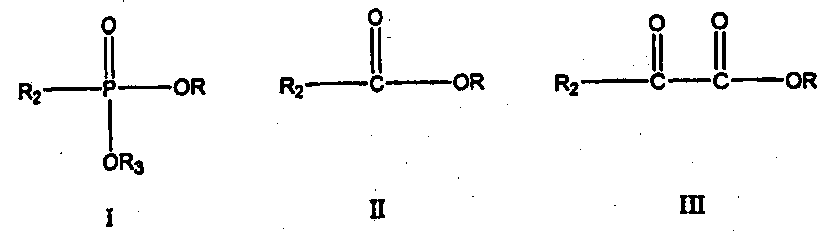 in denen R eine Sterol- oder Stanol-Einheit ist, R₂ eine Ascorbinsäure-Einheit ist und R₃ Wasserstoff oder irgendein Metall, Erdalkalimetall oder Alkalimetall ist; und aller Salze von diesen Strukturen bei der Herstellung einer Zusammensetzung zur Verwendung bei einer Gewichtskontrollmethode bei einem Tier.

2. Verwendung nach Anspruch 1, bei der das Tier ein Mensch ist.

3. Verwendung nach Anspruch 1, bei der das Sterol ausgewählt ist aus der Gruppe bestehend aus Sitosterol, Campesterol, Stigmasterol, Brassicasterol, Desmosterol, Chalinosterol, Poriferasterol, Clionasterol, Ergosterol, Coprosterol, Codisterol, Isofucosterol, Fucosterol, Clerosterol, Nervisterol, Lathosterol, Stellasterol, Spinasterol, Chondrillasterol, Peposterol, Avenasterol, Isoavenasterol, Fecosterol, Pollinastasterol und Cholesterol.

4. Verwendung nach Anspruch 1, bei der das Stanol ausgewählt ist aus der Gruppe bestehend aus allen gesättigten oder hydrierten Sterolen.

5. Verwendung nach Anspruch 1, bei der das Stanol Sitostanol ist.

6. Verwendung nach Anspruch 1, bei der R₃ ausgewählt ist aus der Gruppe bestehend aus Calcium, Magnesium, Mangan, Kupfer, Zink, Natrium, Kalium und Lithium.

7. Verwendung nach irgendeinem vorhergehenden Anspruch, bei der die Gewichtskontrollmethode zu einer Verringerung der Gewichtszunahme führt.

8. Verwendung nach irgendeinem der Ansprüche 1 bis 6, bei der die Gewichtskontrollmethode zu einer Steigerung der Gewichtsabnahme führt.

9. Verwendung nach irgendeinem vorhergehenden Anspruch, bei der die Zusammensetzung ein Nahrungsmittel oder ein Getränk ist.

10. Verwendung nach irgendeinem vorhergehenden Anspruch, bei der ein für Lebensmittel geeigneter oder pharmazeutisch verträglicher Träger dafür bei der Herstellung der Zusammensetzung verwendet wird.

## Revendications

1. Utilisation d'une ou plusieurs des structures répondant aux formules suivantes : 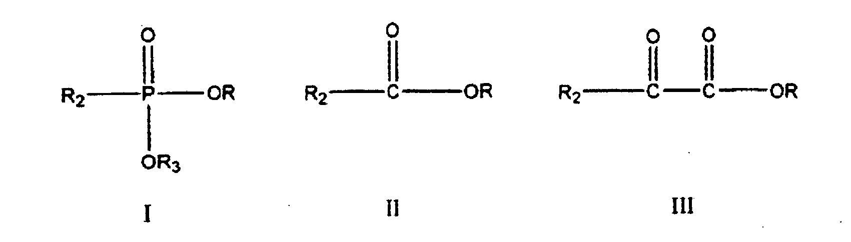 dans lesquelles R est une fraction de stérol ou de stanol, R₂ est une fraction d'acide ascorbique et R₃ est un atome d'hydrogène ou tout métal, métal alcalino-terreux ou métal alcalin ; et tous les sels de ces structures dans la fabrication d'une composition destinée à une utilisation dans un procédé de régulation du poids chez un animal.

2. Utilisation selon la revendication 1, dans laquelle l'animal est un être humain.

3. Utilisation selon la revendication 1, dans laquelle le stérol est choisi dans le groupe constitué par le sitostérol, le campestérol, le stigmastérol, le brassicastérol, le desmostérol, le chalinostérol, le poriférastérol, le clionastérol, l'ergostérol, le coprostérol, le codistérol, l'isofucostérol, le fucostérol, le clérostérol, le nervistérol, le lathostérol, le stellastérol, le spinastérol, le chondrillastérol, le pépostérol, l'avénastérol, l'isoavénastérol, le fécostérol, le pollinastastérol et le cholestérol.

4. Utilisation selon la revendication 1, dans laquelle le stanol est choisi dans le groupe constitué par tous les stérols saturés ou hydrogénés.

5. Utilisation selon la revendication 1, dans laquelle le stanol est le sitostanol.

6. Utilisation selon la revendication 1, dans laquelle R₃ est choisi dans le groupe constitué par le calcium, le magnésium, le manganèse, le cuivre, le zinc, le sodium, le potassium et le lithium.

7. Utilisation selon toutes revendications précédentes, dans laquelle le procédé de régulation du poids aboutit à une diminution du gain de poids.

8. Utilisation selon l'une quelconque des revendications 1 à 6, dans laquelle le procédé de régulation du poids aboutit à une augmentation en perte de poids.

9. Utilisation selon l'une quelconque des revendications précédentes, dans laquelle la composition est un comestible ou une boisson.

10. Utilisation selon l'une quelconque des revendications précédentes, dans laquelle un véhicule de qualité alimentaire ou pharmaceutiquement acceptable pour celle-ci est utilisé dans la fabrication de la composition.
